# EUROPEAN PATENT SPECIFICATION

(11) **EP 2 887 874 B1**
(45) Date of publication and mention of the grant of the patent: **09.11.2016**
(21) Application number: 13783987.4
(22) Date of filing: 09.08.2013
(51) Int. Cl.: A61B 6/03, A61B 6/00, A61B 5/055, G01R 33/48, G01R 33/56, G01T 1/16, G01R 33/565

(54) **MR RECEIVE COIL LOCALIZATION AND MR-BASED ATTENUATION CORRECTION**
MR-EMPFANGSSPULENLOKALISIERUNG UND MR-BASIERTE DÄMPFUNGSKORREKTUR
LOCALISATION DE BOBINE DE RÉCEPTION DE RÉSONANCE MAGNÉTIQUE ET CORRECTION D'ATTÉNUATION BASÉE SUR LA RÉSONANCE MAGNÉTIQUE

(30) Priority: 21.08.2012 US 201261691287 P
(43) Date of publication of application: 01.07.2015
(73) Proprietor: Koninklijke Philips N.V., 5656 AE Eindhoven (NL); Philips GmbH, 20099 Hamburg (DE)
(72) Inventor: STEHNING, Christian, 5656 AE Eindhoven (NL); RENISCH, Steffen, 5656 AE Eindhoven (NL); DAHNKE, Hannes, 5656 AE Eindhoven (NL); HU, Zhiqiang, 5656 AE Eindhoven (NL)
(74) Representative: Cohen, Julius Simon
(86) International application number: PCT/IB2013/056522
(87) International publication number: WO 2014/060863

(56) References cited:
- M. HOFMANN ET AL: "MRI-Based Attenuation Correction for Whole-Body PET/MRI: Quantitative Evaluation of Segmentation- and Atlas-Based Methods", THE JOURNAL OF NUCLEAR MEDICINE, vol. 52, no. 9, 1 September 2011 (2011-09-01), pages 1392-1399, XP055034130, ISSN: 0161-5505, DOI: 10.2967/jnumed.110.078949
- E Mark Haacke ET AL: "Establishing a baseline phase behavior in magnetic resonance imaging to determine normal vs. abnormal iron content in the brain", Journal of magnetic resonance imaging : JMRI, 1 August 2007 (2007-08-01), pages 256-264, XP055096151, United States DOI: 10.1002/jmri.20987 Retrieved from the Internet: URL:http://www.ncbi.nlm.nih.gov/pubmed/176 54738
- BARTUSEK K ET AL: "Magnetic field mapping around metal implants using an asymmetric spin-echo MRI sequence", MEASUREMENT SCIENCE AND TECHNOLOGY, IOP, BRISTOL, GB, vol. 17, no. 12, 1 December 2006 (2006-12-01), pages 3293-3300, XP020103308, ISSN: 0957-0233, DOI: 10.1088/0957-0233/17/12/015

## Description

The present application relates generally to medical imaging. It finds particular application in conjunction with magnetic resonance (MR)-based attenuation correction for nuclear medicine (NM), and will be described with particular reference thereto. However, it is to be understood that it also finds application in other usage scenarios and is not necessarily limited to the aforementioned application.

In NM imaging modalities, such as positron emission tomography (PET) and single-photon emission computed tomography (SPECT) systems, attenuation is the loss of detection events because of absorption in the body or scattering out of the detector field of view. Loss of detection events due to attenuation increases image noise, image artifacts, and image distortion. This, in turn, affects downstream applications of images generated using PET or SPECT, such as radiation therapy planning. Attenuation is especially problematic in whole-body PET scans.

One source of attenuation is MR hardware, such as local receive coils, in combined PET/MR systems. The MR hardware typically remains in the emission patch during the PET image acquisition, since it is impracticable to remove the MR hardware without moving the patient. This causes attenuation, which needs to be corrected for. Therefore, accurate knowledge of the MR hardware position is required. Another source of attenuation is metallic implants, such as stents, pacemakers, orthopaedic devices (e.g., hip replacements), survical screws, pins, and the like.

MR-based attenuation correction is one approach for attenuation correction in PET and SPECT systems. An MR scan is employed to identify the different tissue and/or material types within the field of view of a PET or SPECT system. Attenuation values corresponding to the identified tissue and/or material types are assigned to the corresponding regions. An attenuation map is generated from the identified tissue and/or material types and the assigned attenuation values. For example, in "MRI-based attenuation correction for whole-body PET/MRI: quantitative evaluation of segmentation- and atlas-based methods" (M.Hofmann et al., J Nucl Med, 2011, 52, pp. 1392-1399), two algorithms has been proposed for whole-body MRI-based attenuation correction: a basic MR image segmentation algorithm and a method based on atlas registration and pattern recognition. One challenge with MR-based attenuation correction is that metal does not generate useful resonance signals.

The present application provides a new and improved system and method which overcome the above-referenced problems and others.

In accordance with one aspect, a magnetic resonance (MR) system includes a source of one or more MR data sets of an imaging volume. The imaging volume includes one or more of a region of interest (ROI), the ROI including a metal element and/or a local receive coil of the MR scanner. The system further includes at least one processor programmed to at least one of: (1) generate an attenuation, confidence or density map accounting for the metal element; and (2) determine the location of the local receive coil within the imaging volume. The generating includes identification of the metal element within the ROI based on a phase map of the ROI generated from the MR data sets. The determining includes registering a known sensitivity profile of the local receive coil to a sensitivity map of the local receive coil generated from the MR data sets.

In accordance with another aspect, a magnetic resonance (MR) method including generating one or more MR data sets of an imaging volume using an MR scanner. The imaging volume includes one or more of a region of interest (ROI), the ROI including a metal element, and a local receive coil of the MR scanner. The method further includes at least one of: (1) generating an attenuation, confidence or density map accounting for the metal element; and (2) determining the location of the local receive coil within the imaging volume. The generating includes identification of the metal element within the ROI based on a phase map of the ROI generated from the MR data sets. The determining includes registering a known sensitivity profile of the local receive coil to a sensitivity map of the local receive coil generated from the MR data sets.

In accordance with another aspect, a magnetic resonance (MR) system includes an MR scanner generating one or more MR data sets of an imaging volume. The imaging volume includes a region of interest (ROI), the ROI including a metal element. The system further includes at least one processor programmed to determine one or more of the location of the metal element within the imaging volume and the location of a local receive coil within the imaging volume based on the complex MR data or a phase map of the ROI generated from the MR data sets and/or based on registration of a known sensitivity profile of the local receive coil to a sensitivity map of the local receive coil generated from the MR data sets.

One advantage resides in improved magnetic resonance (MR)-based attenuation correction accounting for attenuation of metal.

Another advantage resides in improved image quality in positron emission tomography (PET) and single-photon emission computed tomography (SPECT) systems.

Another advantage resides in improved radiation therapy planning.

Another advantage resides in localization of a local receive coil without the use of trackers and/or the like.

Another advantage resides in detecting and segmenting metal with high spatial resolution.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 illustrates a combined positron emission tomography (PET) and magnetic resonance (MR) system.
FIGURE 2 illustrates a method for calculating an attenuation map which accounts for metal.
FIGURE 3 illustrates the spectrum shift in phase of a voxel due to proximity to metal.
FIGURE 4 illustrates a method for calculating an attenuation map accounting for a local receive coil within the imaging volume.
FIGURE 5 illustrates a radiation therapy system.

The present application provides means to extract the location of magnetic resonance (MR) receive coil hardware from available MR data, such as by detecting and localizing metal in the MR coil using the complex MR image phase. Further, the present application provides means to locate metal, such as the metal in an MR receive coil or other metal in the MR examination region, using the comprex image phase.

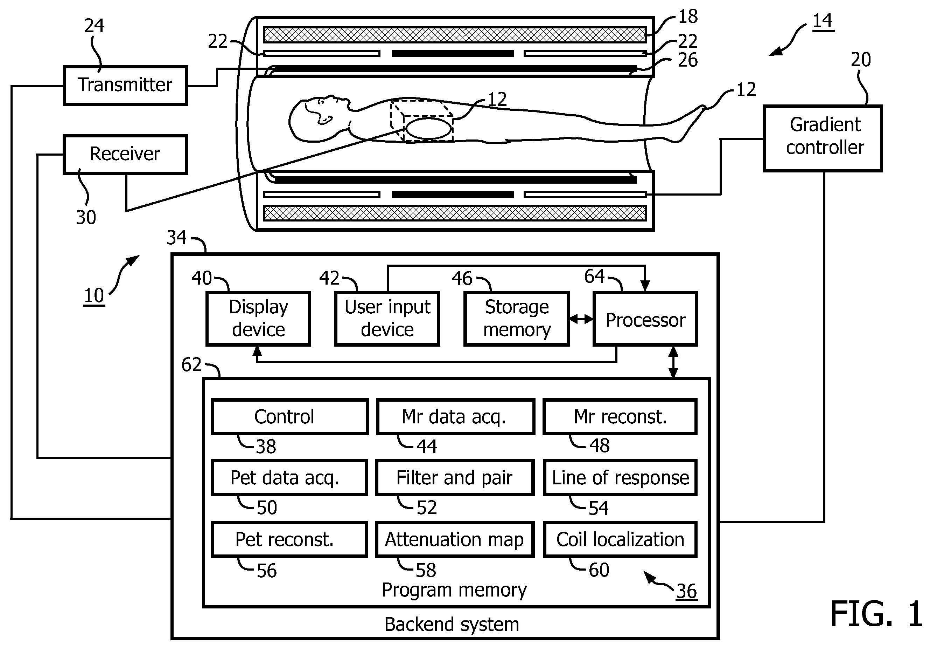

With reference to FIGURE 1, a combined positron emission tomography (PET) and magnetic resonance (MR) system **10** utilizes MR and/or PET to image a region of interest (ROI) of a patient **12.** The system **10** includes a scanner **14** defining an imaging volume **16** (indicated in phantom) sized to accomadate the ROI. The size of the imaging volume **16** can vary depending upon whether MR or PET is employed. A patient support can be employed to support the patient **12** in the scanner **14** and facilitates positioning the ROI in the imaging volume **16.**

For MR imaging, the scanner **14** includes a main magnet **18** that creates a strong, static B₀ magnetic field extending through the imaging volume **16.** The main magnet **18** typically employs superconducting coils to create the static B₀ magnetic field. However, the main magnet **18** can also employ permanent or resistive magnets. Insofar as superconducting coils are employed, the main magnet **18** includes a cooling system, such as a liquid helium cooled cryostat, for the superconducting coils. The strength of the static B₀ magnetic field is commonly one of 0.23 Tesla, 0.5 Tesla, 1.5 Tesla, 3 Tesla, 7 Tesla, and so on in the imaging volume **16,** but other strengths are contemplated.

A gradient controller **20** of the scanner **14** is controlled to superimpose magnetic field gradients, such as x, y and z gradients, on the static B₀ magnetic field in the imaging volume **16** using a plurality of magnetic field gradient coils **22** of the scanner **14.** The magnetic field gradients spatially encode magnetic spins within the imaging volume **16.** Typically, the plurality of magnetic field gradient coils **22** include three separate magnetic field gradient coils spatially encoding in three orthogonal spatial directions.

Further, one or more transmitters **24,** such as a transceiver, of the scanner **14** are controlled to transmit B₁ resonance excitation and manipulation radiofrequency (RF) pulses into the imaging volume **16** with one or more transmit coils, such as a whole body coil **26** and/or a surface coil **28,** of the scanner **14.** The B₁ pulses are typically of short duration and, when taken together with the magnetic field gradients, achieve a selected manipulation of magnetic resonance. For example, the B₁ pulses excite the hydrogen dipoles to resonance and the magnetic field gradients encode spatial information in the frequency and phase of the resonance signal. By adjusting the RF frequencies, resonance can be excited in other dipoles, such as phosphorous, which tend to concentrate in known tissues, such as bones.

One or more receivers **30,** such as a transceiver, of the scanner **14** are controlled to receive spatially encoded magnetic resonance signals from the imaging volume **16** and demodulate the received spatially encoded magnetic resonance signals to MR data sets. The MR data sets include, for example, k-space data trajectories. To receive the spatially encoded magnetic resonance signals, the receivers **30** use one or more receive coils, such as the whole body coil **26** and/or the surface coil **28,** of the scanner **14.** The receivers **30** typically store the MR data sets in a buffer memory.

For PET imaging, the scanner **14** includes a plurality of detector modules **32,** typically solid state detector modules, arranged around the imaging volume **16,** typically in a ring or cylinder. Each of the detector modules **32** includes a two-dimensional array of radiation sensitive elements. The radiation sensitive elements directly or indirectly detect radiation (i.e., gamma photons) from the imaging volume **16** and, based upon the detected radiation, generate gamma event data indicating the energy of detected radiation. Examples of radiation sensitive elements include scintillators with digital or analog silicon photomultipliers (SiPMs), photodiodes, zinc-cadmium telluride (CZT) elements, and the like.

If the SiPMs do not directly detect the radiation, the detector modules **32** typically include one or more scintillators optically coupled to the radiation sensitive elements between the imaging volume **16** and the radiation sensitive elements. When a gamma photon deposits energy in the scintillators, the scintillators scintillate and emit photons (typically light photons) toward the radiation sensitive elements, which the radiation sensitive elements can directly detect. Examples of scintillators include scintillator plates (e.g., sodium iodide crystals), individual scintillation or pixelated crystals (e.g., LYSO, LSO, etc.), and the like.

A backend system **34** of the system **10** images the ROI using the scanner **14.** The backend system **34** is typically remote from the scanner **14** and includes a plurality of modules **36,** discussed hereafter, to perform the imaging of the ROI using the scanner **14.** As will be seen, the backend system **34** advantageously provides improved attenuation correction (AC) for PET imaging which takes into account attenuation caused by metal and local receive coils within the imaging volume **16.**

A control module **38** of the backend system **34** controls overall operation of the backend system **34.** The control module **38** suitably displays a graphical user interface (GUI) to a user of the backend system **34** using a display device **40** of the backend system **34.** Further, the control module **36** suitably allows the operator to interact with the GUI using a user input device **42** of the backend system **34.** For example, the user can interact with the GUI to instruct the backend system **34** to coordinate the imaging of the ROI.

An MR data acquisition module **44** of the backend system **34** performs MR diagnostic scans of the ROI. For each MR diagnostic scan, the data acquisition module **44** controls the transmitters **24** and/or the gradient controller **20** according to scan parameters, such as number of slices, to implement an imaging sequence within the imaging volume **16.** An imaging sequence defines a sequence of B₁ pulses and/or magnetic field gradients that produce spatially encoded MR signals from the imaging volume **16.** Further, the data acquisition module **44** controls the receivers **30** according to scan parameters to acquire spatially encoded MR signals to an MR data set. The MR data set is typically stored in at least one storage memory **46** of the backend system **34.**

In preparing for MR acquisition, the ROI is positioned within the imaging volume **16.** For example, the patient **12** is positioned on the patient support. The surface coil **28** is then positioned on the patient **12** and the patient support moves the ROI into the imaging volume **16.**

An MR reconstruction module **48** of the backend system **34** reconstructs the MR data sets of the MR diagnostic scans into MR images or maps of the ROI. This includes, for each MR signal captured by the MR data sets, spatially decoding the spatial encoding by the magnetic field gradients to ascertain a property of the MR signal from each spatial region, such as a pixel or voxel. The intensity or magnitude of the MR signal is commonly ascertained, but other properties related to phase, relaxation time, magnetization transfer, and the like can also be ascertained. The MR images or maps are typically stored in the storage memory **46.**

A PET data acquisition module **50** of the backend system **34** performs PET diagnostic scans of the ROI. This includes, for each PET diagnostic scan, acquiring gamma event data for the ROI from the detector modules **32** to a PET data set. The gamma event data is acquired over a data acquisition period of a predetermined length, such as several milliseconds. The PET data set includes gamma event data for all the scintillation events detected by the detector modules **32** during the data acquisition period. The PET data set is typically stored in the storage memory **46.**

In preparing for PET acquisition, the ROI is injected with one or more radioisotopes. Examples of such radioisotopes include, but are not limited to, F 18, Rb 82, C 11, O 15, and the like. The radioisotopes can be combined and injected with radioligands to create a radiopharmaceutical that binds to or is preferentially absorbed by specific types of tissue. Further, the ROI is positioned in the imaging volume **16.** For example, the patient **12** is positioned on the patient support and the patient support moves the ROI into the imaging volume **16.**

A filtering and pairing module 52 of the backend system **34** filters invalid gamma events from the PET data sets. Invalid gamma events are typically gamma events with detected energies other than about 511 keV. Further, corresponding pairs of gamma events are identified based on time stamps associated with detected gamma events. Those gamma events seperated by less than a predetermined time are paired. Thereafter, a line of response (LOR) module **54** of the backend system **34,** for each identified pair of gamma events, processes the spatial information pertaining to the two gamma events to identify a spatial LOR connecting the two gamma event detections.

A PET reconstruction module **56** of the backend system **34** reconstructs the PET data sets of the PET diagnostic scans into PET images of the ROI using the LORs identified by the LOR module **54.** Namely, for each PET data set, the corresponding LORs are reconstructed into a PET image with a PET reconstruction algorithm. Any number of well-known PET reconstruction algorithms can be employed. The PET reconstruction algorithm suitably takes into account an attenuation map stored in the storage memory **46.** The PET images are typically stored in the storage memory **46.**

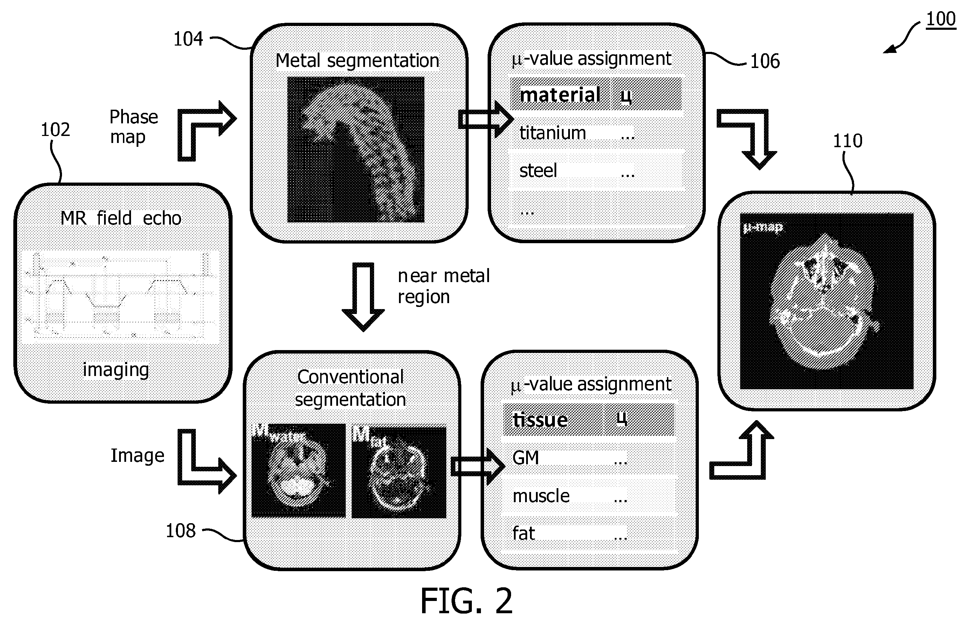

An attenuation map module **58** of the backend system **34** calculates an attenuation map for PET imaging. The attenuation map can take in to account metal and/or one or more local receive coils, as described in detail hereafter. With further reference to FIGURE 2, a block diagram of method **100** for calculating an attenuation map which accounts for metal is provided. The attenuation map module **58** can be configured to perform the method **100,** which includes performing **102** an MR scan of the ROI using the MR data acquisition module **44** to generate an MR data set. The MR scan is suitably performed using an MR field echo imaging sequence, such as DIXON.

A phase map of the ROI is generated from the MR data set using the MR reconstruction module **48.** Further, metal segmentation is performed **104** to identify metallic regions in the phase map. As noted above, metal does not image directly. Hence, metallic regions cannot be directly identified. However, the behaviour of the complex MR image phase in the vicinity of metal allows detecting and segmenting metallic regions. Namely, metal in the imaging volume **16** results in a high local susceptibility effect, which manifests itself in an alteration of the linear image phase around the metal.

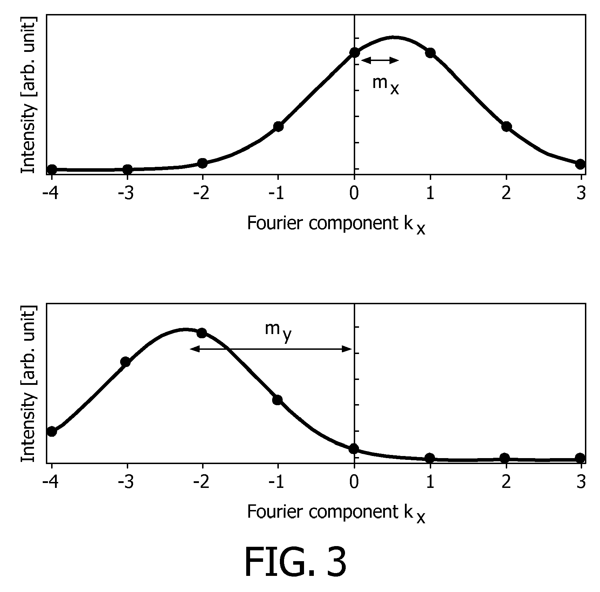

A method to detect these shifts and reconstruct the susceptibility gradient (magnitude and direction) is based on a Fourier analysis of voxels in the vicinity of metal. As illustrated in FIGURE 3, in the vicinity of metal, the spectrum peak is shifted (mₓ, my). The alignment of included voxels allows the susceptibility gradient direction (pointing towards metal) to be extracted. Further, the shift reflects the susceptibility gradient magnitude. Hence, metallic regions can be indirectly identified and segmented based on a Fourier analysis of the phase map.

As an alternative to identifying metallic regions using a phase map generated from the MR data set, an image of the ROI can be generated using a different imaging modality capable of imaging metal. The image can then be used to identify metallic regions in the ROI, and these identified metallic regions can be mapped to the coordinate frame of the MR scan using a registration algorithm.

After identifying metallic regions, the regions containing metal are labeled as "metal" and the nearby dark regions are labeled as "near metal". The near metal regions are those with shifts exceeding a predetermined threshold, and the metal regions are those surrounded by the near metal regions. Using the identifed regions, attenuation values (µ) are assigned 106 to the metal and near metal regions. A confidence map for the ROI can additionally or alternatively be generated indicating a confidence in PET image quality, where the near metal and metal regions are assigned low and lower confidence levels, respectively.

The attenuation values (µ) for the metallic regions can be derived from user input. For example, for each metallic region, the user can specify the metal type using the user input device **42.** The value can then be used to look up a known attenuation value for the specified metal. As another example, for each metallic region, the user can specify the attenuation value using the user input device **42.** As an alternative to deriving the attenuation values from user input, the metallic regions can be assigned a predetermined value approximating the attenuation of metal. The attenuation values for the near metal regions are typically assigned the attenuation values of the neighboring tissue and/or material other than metal, which are determined below. Other approaches are, however, contemplated.

In addition to generating the phase map of the ROI, one or more images of the ROI are generated from the MR data set using the MR reconstruction module **48.** For example, an image for fat and/or water can be generated. Further, tissue and/or material regions, which are outside the metal and near metal regions, are are identified using conventional MR-based tissue segmentation **108.** The attenuation values (µ) for these regions can be derived from user input, as described above. However, these regions are typically assigned attenuation values looked up from a lookup table based on tissue and/or material type. Namely, the lookup table includes the known attenuation values for known tissue and/or material types which can be identified using MR.

Once the tissue and metal attenuation values are determined, they are combined **110** into an attenuation map, which maps between tissue and/or material type and attenuation value. The attenuation map is typically stored in the storage memory **46.** The PET reconstruction processor **56** then employs the attenuation map for attenuation when reconstructing a PET data set into an image.

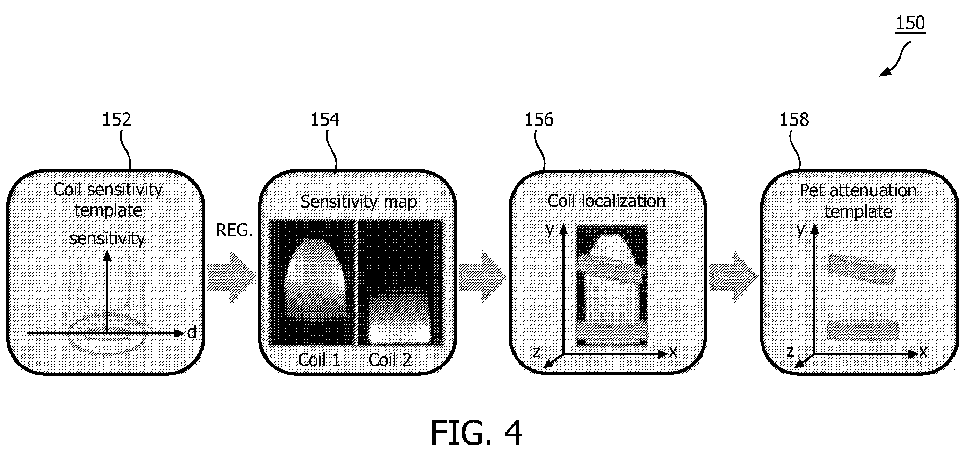

With further reference to FIGURE 4, a method **150** for calculating an attenuation map accounting for a local receive coil within the imaging volume **16,** such as the surface coil **28,** is provided. The attenuation map module **58** can be configured to perform the method **150** for each of the local receive coils. The method **150** includes registering a template **152** of a known, typical sensitivity profile of the local receive coil to an actually measured coil sensitivity map **154** in order to obtain the coil position in 3D image space **156.** A non-rigid registration procedure can be employed to cope with flexible receive coils. The coil sensitivity map represents a measurement of a local coil sensitivity distribution for each individual coil element, and is commonly acquired to accelerate MR data acquisition using sensitivity encoding (SENSE). Suitably, the data acquisition module **44** is employed to acquire coil sensitivity map as part of an MR data set.

After registering the template **152,** a coil-specific attenuation map template **158** with the known coil material absorption values is fit into an attenuation map at the appropriate position. For example, the attenuation map can be fit in to an attenuation map generated using the method **100** of FIGURE 2.

The relative position of the whole body coil elements and the PET detectors is fixed during manufacturing. The attenuation values to correct for whole body coil attenuation are precalculated and added to the attenuation map.

In another embodiment, the MR field echo sequence is performed with the whole body coil 26 and the metallic local coil elements are located from the phase gradients in polymeric material that encapsulates the metal elements.

Referring to FIGURE 1, each of the plurality of modules **36** can be embodied by processor executable instructions, circuitry (i.e., processor independent), or a combination of the two. The processor executable instructions are stored on at least one program memory **62** of the backend system **34** and executed by at least one processor **64** of the backend system **34.** As illustrated, the plurality of modules **36** are embodied by processor executable instructions. However, as is to be appreciated, variations are contemplated. For example, the filtering and pairing module **52** can be circuitry.

While the foregoing discussion was specific to a combined MR and PET system, it is to be appreciated that the foregoing discussion finds application to any form of nuclear medicine (i.e., PET, single-photon emission computed tomography (SPECT), radiation therapy, and so on), where MR image data is available and the presence of metal needs to be taken into account during an attenuation correction process. Further, it is to be appreciated that a combined MR and nuclear medicine system is not necessary.

For example, the system **10** can be employed for SPECT imaging through application of a SPECT reconstruction algorithm to a data set acquired from the detector modules **32,** since SPECT operates similar to PET. As another example, the system **10** can be employed for radiation therapy through addition of a radiation therapy system and, optionally, removal of the PET hardware. PET images corrected for attenuation using an attenuation map generated above can be combined with segmented MR images and employed for therapy planning. Alternatively, instead of generating an attenuation map, as described above, a density map can be generated. The difference being that instead of using attenuation values, density values are employed. The density map can then be employed for planning.

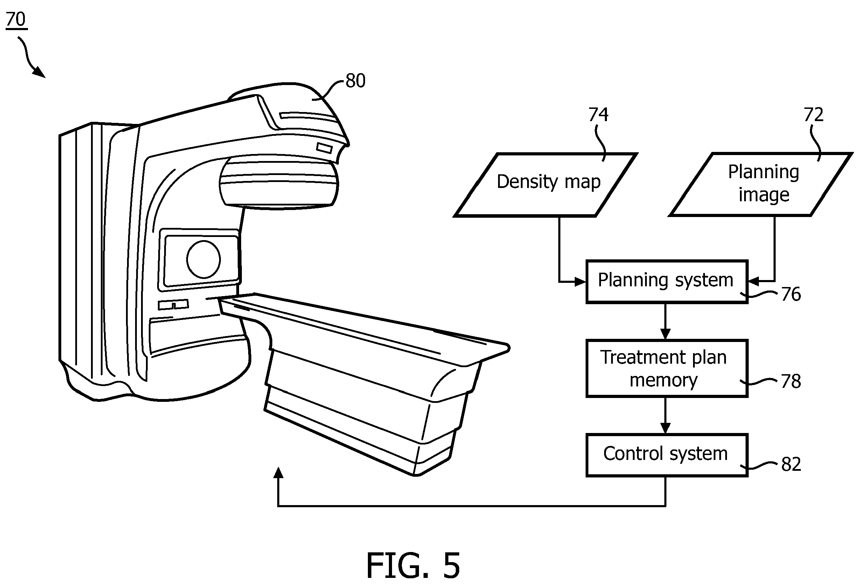

With reference to FIGURE 5, a radiation therapy system **70** which can be included in the system **10** is provided. The radiation therapy system **70** receives a planning image **72** of the ROI and, optionally, a density map **74,** which indicates attenuation of the therapy beam as it passes through the various tissues, metal or other structures. Insofar as the radiation therapy system **70** does not receive the density map **74,** the planning image **72** is suitably a PET image attenution corrected as described above. Insofar as the radiation therapy system **70** receives the density map, the density map **74** is suitably registered to the planning image **72.**

A planning system **76** of the therapy system **70** identifies targets and, optionally, organs at risk in the planning image **72.** Further, the planning system **76** receives plan parameters from a user. Based at least on the identified regions within the planning image **72** and the plan parameters, the planning system **76** generates a treatment plan. Where the radiation therapy system **70** receives the density map **74,** the generation of the treatment plan is further based on the density map **74.** The treatment plan is suitably stored in a treatment plan memory **78.**

At a scheduled day and time for a therapy session, a therapy delivery apparatus **80** of the radiation therapy system **70** delivers therapy. The therapy, such as ablation therapy and/or brachytherapy, can include radiation involving one or more of x-rays, gamma rays, protons, HIFU, and the like. Suitably, the therapy delivery apparatus **80** is controlled by a control system **82** in accordance with the treatment plan.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), an FPGA, and the like; a controller includes: (1) a processor and a memory, the processor executing computer executable instructions on the memory embodying the functionality of the controller; or (2) analog and/or digital hardware; a user input device includes one or more of a mouse, a keyboard, a touch screen display, one or more buttons, one or more switches, one or more toggles, voice recognition engines, and the like; a database includes one or more memories; and a display device includes one or more of a LCD display, an LED display, a plasma display, a projection display, a touch screen display, and the like.

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

## Claims

1. A magnetic resonance (MR) system **(10)** comprising:
a source **(14, 46)** of one or more MR data sets of an imaging volume **(16),** the imaging volume **(16)** including one or more of a region of interest (ROI), the ROI including a metal element of the MR scanner **(14);** and,
at least one processor **(64)** programmed to:
generate an attenuation, confidence or density map accounting for the metal element, **characterised in that** the generating includes identification of metallic regions within the ROI based on a phase map of the ROI generated from the MR data sets.

2. The MR system **(10)** according to claim 1, wherein the processor **(64)** is further programmed to:
perform a Fourier analysis of the phase map to identify regions of the ROI with the metal element induced phase shift from the linear phase of the phase map, wherein the metal element corresponds to one or more regions surrounded by the identified metal element induced phase shift regions.

3. The MR system **(10)** according to claim 2, wherein the processor is further programmed to:
assign the identified regions attenuation or density values of neighboring regions of the ROI which do not have the metal element induced phase shift.

4. The MR system **(10)** according to either one of claims 2 and 3, wherein the processor is further programmed to:
assign regions of the ROI confidence values, wherein the regions corresponding to the metal element are assigned confidence values less than confidence values of the identified regions, which are assigned confidence values less than regions of the ROI which do not have the metal element induced phase shift and which do not correspond to the metal element.

5. The MR system **(10)** according to any one of claims 1-4, wherein the registering uses a non-rigid registration algorithm.

6. The MR system **(10)** according to any one of claims 1-5, further including:
a radiation therapy planning system **(76)** which generates a treatment plan using:
the attenuation, confidence or density map.

7. The MR system **(10)** according to any one of claims 1-6, further including:
a positron emission tomography (PET) or a single-photon emission computed tomography (SPECT) system **(10)** generating an image of the ROI, the image corrected for attenuation using:
the attenuation, confidence or density map, wherein the attenuation, confidence or density map is an attenuation map.

8. The MR system **(10)** according to claim 7, wherein the attenuation corrected image of the ROI is combined with an MR image of the ROI generated from the MR data sets.

9. A magnetic resonance (MR) method comprising:
generating one or more MR data sets of an imaging volume **(16)** using an MR scanner **(14),** the imaging volume **(16)** including one or more of a region of interest (ROI), the ROI including a metal element; and,
generating an attenuation, confidence or density map accounting for the metal element, **characterised in that** the generating includes identification of metallic regions within the ROI based on a phase map of the ROI generated from the MR data sets.

10. The MR method according to claim 9, wherein the identification includes:
performing a Fourier analysis of the phase map to identify regions of the ROI with a phase shift from the linear phase of the phase map, wherein the metal element corresponds to one or more regions surrounded by the identified regions.

11. The MR method according to any one of claims 9 -10, further including:
generating a radiation therapy treatment plan using:
the attenuation, confidence or density map.

12. The MR method according to any one of claims 9 -11 further including:
generating an image of the ROI using positron emission tomography (PET) or single-photon emission computed tomography (SPECT), the image corrected for attenuation using:
the attenuation, confidence or density map.

13. At least one processor **(64)** programmed to perform the method according to any one of claims 9-12.

14. A non-transitory computer readable medium **(62)** carrying software which controls one or more processors **(64)** to perform the method according to any one of claims 9-12.

## Patentansprüche

1. Magnetresonanz- (MR) System (10), das Folgendes umfasst:
eine Quelle (14, 46) von einem oder mehreren MR-Datensätzen eines Bildgebungsvolumens (16), wobei das Bildgebungsvolumen (16) eine oder mehrere interessierende Regionen (region of interest, ROI) umfasst, wobei die interessierende Region ein Metallelement des MR-Scanners (14) umfasst; und
mindestens einen Prozessor (64), der programmiert ist zum
Erzeugen einer Abschwächungs-, Konfidenz- oder Dichtekarte, in der das Metallelement berücksichtigt wird, **dadurch gekennzeichnet, dass** das Erzeugen das Identifizieren von metallischen Regionen innerhalb der interessierenden Region basierend auf einer Phasenkarte der interessierenden Region umfasst, die anhand der MR-Datensätze erzeugt wird.

2. MR-System (10) nach Anspruch 1, wobei der Prozessor (64) weiterhin programmiert ist zum:
Durchführen einer Fourier-Analyse der Phasenkarte, um Regionen der interessierenden Region mit der durch das Metallelement induzierten Phasenverschiebung von der linearen Phase der Phasenkarte zu identifizieren, wobei das Metallelement einer oder mehreren Regionen entspricht, die durch die identifizierten Regionen mit einer durch das Metallelement induzierten Phasenverschiebung umgeben sind.

3. MR-System (10) nach Anspruch 2, wobei der Prozessor weiterhin programmiert ist zum:
Zuweisen von Abschwächungs- oder Dichtewerten von benachbarten Regionen der interessierenden Region, die keine durch das Metallelement induzierte Phasenverschiebung aufweisen, zu den identifizierten Regionen.

4. MR-System (10) nach einem der Ansprüche 2 und 3, wobei der Prozessor weiterhin programmiert ist zum:
Zuweisen von Konfidenzwerten zu Regionen der interessierenden Region, wobei den Regionen, die dem Metallelement entsprechen, Konfidenzwerte zugewiesen werden, die geringer sind als die Konfidenzwerte der identifizierten Regionen, denen Konfidenzwerte zugewiesen werden, die geringer sind für die Regionen der interessierenden Region, die keine durch das Metallelement induzierte Phasenverschiebung aufweisen und die nicht dem Metallelement entsprechen.

5. MR-System (10) nach einem der Ansprüche 1 bis 4, wobei für das Registrieren ein nicht-starrer Registrierungsalgorithmus verwendet wird.

6. MR-System (10) nach einem der Ansprüche 1 bis 5, das weiterhin Folgendes umfasst:
ein Strahlentherapie-Planungssystem (76), das einen Behandlungsplan unter Verwendung der Abschwächungs-, Konfidenz- oder Dichtekarte erzeugt.

7. MR-System (10) nach einem der Ansprüche 1 bis 6, das weiterhin Folgendes umfasst:
ein Positronenemissionstomographie- (PET) oder ein Einzelphotonenemissionscomputertomographie- (SPECT) System (10), das ein Bild einer interessierenden Region erzeugt, wobei das Bild bezüglich der Abschwächung korrigiert wird unter Verwendung:
der Abschwächungs-, Konfidenz- oder Dichtekarte, wobei die Abschwächungs-, Konfidenz- oder Dichtekarte eine Abschwächungskarte ist.

8. MR-System (10) nach Anspruch 7, wobei das bezüglich der Abschwächung korrigierte Bild der interessierenden Region mit einem MR-Bild der interessierenden Region kombiniert wird, das anhand der MR-Datensätze erzeugt wird.

9. Magnetresonanz- (MR) Verfahren, das Folgendes umfasst:
Erzeugen von einem oder mehreren MR-Datensätzen eines Bildgebungsvolumens (16) mit Hilfe eines MR-Scanners (14), wobei das Bildgebungsvolumen (16) eine oder mehrere interessierende Regionen (region of interest, ROI) umfasst, wobei die interessierende Region ein Metallelement umfasst; und
Erzeugen einer Abschwächungs-, Konfidenz- oder Dichtekarte, in der das Metallelement berücksichtigt wird, **dadurch gekennzeichnet, dass** das Erzeugen das Identifizieren von metallischen Regionen innerhalb der interessierenden Region basierend auf einer Phasenkarte der interessierenden Region umfasst, die anhand der MR-Datensätze erzeugt wird.

10. MR-Verfahren nach Anspruch 9, wobei das Identifizieren Folgendes umfasst:
Durchführen einer Fourier-Analyse der Phasenkarte, um Regionen der interessierenden Region mit einer Phasenverschiebung von der linearen Phase der Phasenkarte zu identifizieren, wobei das Metallelement einer oder mehreren Regionen entspricht, die durch die identifizierten Regionen umgeben sind.

11. MR-Verfahren nach einem der Ansprüche 9 bis 10, das weiterhin Folgendes umfasst:
Erzeugen eines Strahlentherapie-Behandlungsplans unter Verwendung der Abschwächungs-, Konfidenz- oder Dichtekarte.

12. MR-Verfahren nach einem der Ansprüche 9 bis 11, das weiterhin Folgendes umfasst:
Erzeugen eines Bilds der interessierenden Region mit Hilfe eines Positronenemissionstomographie- (PET) oder eines Einzelphotonenemissionscomputertomographie- (SPECT) Systems, wobei das Bild bezüglich der Abschwächung korrigiert wird unter Verwendung:
der Abschwächungs-, Konfidenz- oder Dichtekarte.

13. Mindestens ein Prozessor (64), der programmiert ist, um das Verfahren nach einem der Ansprüche 9 bis 12 durchzuführen.

14. Nichtflüchtiges computerlesbares Medium (62) mit Software, die einen oder mehrere Prozessoren (64) steuert, um das Verfahren nach einem der Ansprüche 9 bis 12 durchzuführen.

## Revendications

1. Système de résonance magnétique (MR) (10) comprenant :
une source (14, 46) d'un ou de plusieurs ensembles de données MR d'un volume d'imagerie (16), le volume d'imagerie (16) comprenant une ou plusieurs régions d'intérêt (ROI), les ROI comprenant un élément métallique de l'appareil d'imagerie par résonance magnétique (14) ; et
au moins un processeur (64) programmé pour :
générer une carte d'atténuation, de confiance ou de densité représentant l'élément métallique, **caractérisé en ce que** la génération comprend l'identification de régions métalliques à l'intérieur des ROI sur la base d'une carte de phase des ROI générée à partir des ensembles de données MR.

2. Système MR (10) selon la revendication 1, dans lequel le processeur (64) est en outre programmé pour :
réaliser une analyse de Fourier de la carte de phase pour identifier des régions des ROI présentant le décalage de phase induit par l'élément métallique à partir de la phase linéaire de la carte de phase, dans lequel l'élément métallique correspond à une ou plusieurs régions entourées par les régions de décalage de phase induit par l'élément métallique identifiées.

3. Système MR (10) selon la revendication 2, dans lequel le processeur est en outre programmé pour :
attribuer les valeurs d'atténuation ou de densité de régions identifiées de régions voisines des ROI qui ne présentent pas le décalage de phase induit par l'élément métallique.

4. Système MR (10) selon l'une quelconque des revendications 2 et 3, dans lequel le processeur est en outre programmé pour :
attribuer des régions de valeurs de confiance de ROI, dans lequel les régions correspondant à l'élément métallique sont des valeurs de confiance attribuées inférieures aux valeurs de confiance des régions identifiées, qui sont des valeurs de confiance attribuées inférieures à des régions des ROI qui ne présentent pas le décalage de phase induit par l'élément métallique et qui ne correspondent pas à l'élément métallique.

5. Système MR (10) selon l'une quelconque des revendications 1-4, dans lequel l'enregistrement utilise un algorithme d'enregistrement non rigide.

6. Système MR (10) selon l'une quelconque des revendications 1-5, comprenant en outre :
un système de planification de radiothérapie (76) qui génère un plan de traitement au moyen de :
la carte d'atténuation, de confiance ou de densité.

7. Système MR (10) selon l'une quelconque des revendications 1-6, comprenant en outre :
un système de tomographie par émission de positrons (PET) ou de tomographie d'émission monophotonique assistée par ordinateur (SPECT) (10) générant une image des ROI, l'image corrigée pour l'atténuation utilisant :
la carte d'atténuation, de confiance ou de densité, dans lequel la carte d'atténuation, de confiance ou de densité est une carte d'atténuation.

8. Système MR (10) selon la revendication 7, dans lequel l'image à atténuation corrigée des ROI est combinée à une image MR des ROI générée à partir des ensembles de données MR.

9. Procédé de résonance magnétique (MR) comprenant :
la génération d'un ou de plusieurs ensembles de données MR d'un volume d'imagerie (16) au moyen d'un appareil d'imagerie par résonance magnétique (14), le volume d'imagerie (16) comprenant une ou plusieurs régions d'intérêt (ROI), les ROI comprenant un élément métallique ; et,
la génération d'une carte d'atténuation, de confiance ou de densité représentant l'élément métallique, **caractérisé en ce que** la génération comprend l'identification de régions métalliques à l'intérieur des ROI sur la base d'une carte de phase des ROI générée à partir des ensembles de données MR.

10. Procédé MR selon la revendication 9, dans lequel l'identification comprend :
la réalisation d'une analyse de Fourier de la carte de phase pour identifier des régions des ROI présentant un décalage de phase à partir de la phase linéaire de la carte de phase, dans lequel l'élément métallique correspond à une ou plusieurs régions entourées par les régions identifiées.

11. Procédé MR selon l'une quelconque des revendications 9-10, comprenant en outre :
la génération d'un plan de traitement de radiothérapie au moyen de :
la carte d'atténuation, de confiance ou de densité.

12. Procédé MR selon l'une quelconque des revendications 9-11, comprenant en outre :
la génération d'une image des ROI au moyen d'une tomographie par émission de positrons (PET) ou d'une tomographie d'émission monophotonique assistée par ordinateur (SPECT), l'image corrigée pour l'atténuation utilisant :
la carte d'atténuation, de confiance ou de densité.

13. Au moins un processeur (64) programmé pour réaliser le procédé selon l'une quelconque des revendications 9-12.

14. Support lisible par un ordinateur non transitoire (62) supportant un logiciel qui commande un ou plusieurs processeurs (64) pour réaliser le procédé selon l'une quelconque des revendications 9-12.
